(19) Europäisches Patentamt

European Patent Office

Office européen des brevets

(11) Veröffentlichungsnummer: **0 252 276 B1**

(12) **EUROPÄISCHE PATENTSCHRIFT**

(45) Veröffentlichungstag der Patentschrift: **12.08.92**

(21) Anmeldenummer: **87107879.6**

(22) Anmeldetag: **01.06.87**

(51) Int. Cl.⁵: **A01N 43/16**, //(A01N43/16, 37:40,37:36,37:10,37:06,31:04, 31:02)

(54) **Desinfektions- und Reinigungsmittel.**

(30) Priorität: **09.06.86 DE 3619375**

(43) Veröffentlichungstag der Anmeldung:
**13.01.88 Patentblatt 88/02**

(45) Bekanntmachung des Hinweises auf die
Patenterteilung:
**12.08.92 Patentblatt 92/33**

(84) Benannte Vertragsstaaten:
**AT BE CH DE ES FR GB IT LI NL SE**

(56) Entgegenhaltungen:
**DE-A- 2 821 585**

**CENTRAL PATENTS INDEX, BASIC ABSTRACTS JOURNAL AGDOC, Woche 8526, 21. August 1985, Zusammenfassung Nr. 85-156869/26, Derwent Publications Ltd, London, GB; & JP-A-60 089 402 (YOSHITOMI PHARM. IND.) 20-05-1985**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Lehmann, Rudolf, Dr.**
**Schnugsheide 2**
**W-5653 Leichlingen(DE)**
Erfinder: **Hachmann, Klaus, Dr.**
**Am Eichelkamp 14**
**W-4010 Hilden(DE)**
Erfinder: **Biermann, Manfred, Dr.**
**Stooterstrasse 18**
**W-4330 Mülheim/Ruhr(DE)**
Erfinder: **Schnegelberger, Harald, Dr.**
**Stieglitzweg 2**
**W-5653 Leichlingen(DE)**

EP 0 252 276 B1

## Beschreibung

Die Erfindung betrifft die Verwendung von Alkylglycosiden als Potenzierungsmittel in alkohol-oder carbonsäurehaltigen antiseptischen Mitteln sowie derartige, Alkohole oder Carbonsäure enthaltende Desinfektions-und Reinigungsmittel mit verstärkter bakterizider Wirkung.

Alkylglycoside, ihre Herstellung und ihre Verwendung, insbesondere als Tenside, sind seit langer Zeit bekannt. Verwiesen wird beispielsweise auf die US-PSen 3 839 318, 3 707 535 und 3 547 828, die DE-OSen 19 05 523, 19 43 689, 20 36 472 und 30 01 064 sowie die EP-OS 0 077 167. Die Herstellung von Alkylglycosiden erfolgt insbesondere durch Umsetzung von Glucose oder Oligosacchariden mit Alkoholen mit 8 bis 25 C-Atomen im Alkylrest. Es entstehen auf diesem Wege bio logisch abbaubare obenflächenaktive Stoffe, die vielseitige Verwendung finden können.

Bei der Untersuchung der mikrobiologischen, insbesondere antimikrobiellen Eigenschaften von Alkylglycosiden zeigte sich, daß diese selbst bei hohen Anwendungskonzentrationen bis zu 10 000 ppm alleine keine wesentlichen antimikrobiellen Wirkungen aufweisen. Auch ihre Kombination mit quartären Ammoniumverbindungen führt zu keinen bemerkenswerten Effekten, obwohl quartäre Ammoniumverbindungen als solche bakterizide Wirkung haben. Der kombinierte Einsatz von Alkylglycosiden und quartären Ammoniumverbindungen, wie er beispielsweise in der US-PS 3 547 828 beschrieben ist, führt zu keinen unerwarteten technischen Effekten.

Die antimikrobielle Wirkung von Alkoholen bzw. Carbonsäuren ist ebenfalls seit längerer Zeit bekannt. Im begrenzten Bereich findet sie auch in der Praxis Anwendung. Die Verwendung von Alkoholen bzw. Carbonsäuren in antimikrobiell wirkenden Mitteln hat jedoch zahlreiche, in der Praxis immer wieder festgestellte Nachteile. So führt beispielsweise der relativ hohe Dampfdruck der in den antimikrobiell wirkenden Mitteln verwendeten Alkohole zu einer Gefährdung der mit derartigen Mitteln Arbeitenden. Außerdem verarmen die Mittel bei längerer Lagerzeit, insbesondere unter erhöhten Temperaturen, an dem antimikrobiell wirkenden Alkohol. Ergänzend ist als Nachteil immer wieder festgestellt worden, daß Alkohole erst in relativ hohen Konzentrationen eine befriedigende antimikrobielle Wirkung entfalten: In der Regel werden Alkoholkonzentrationen im Bereich zwischen 20 und 30 % angetroffen. Andererseits führt die Verwendung von Carbonsäuren in antimikrobiell wirkenden Mitteln immer wieder zu unzumutbarer Geruchsbelästigung, wenn sie in den für eine mikrobielle Wirksamkeit der Mittel erforderlichen Konzentration eingesetzt werden müssen.

Die Erfindung stellte sich somit die Aufgabe, einfache, biologisch leicht abbaubare chemische Verbindungen zu finden, deren Kombination mit an sich bekannten, antimikrobiell wirkenden Verbindungen zu einer Wirkungsverstärkung der Antimikrobika führt, so daß deren Einsatzkonzentration in antimikrobiell wirkenden Mitteln verringert und damit die oben aufgezählten Nachteile vermindert, wenn nicht gar ganz beseitigt werden können. Auch aus ökonomischen Gründen war es sinnvoll, nach einfachen, biologisch abbaubaren Substanzen zu suchen, die in Kombination mit den genannten antimikrobiellen Verbindungen zu einer synergistischen Leistungssteigerung dieser Wirkstoffe, insbesondere bezüglich bestimmter mikrobizider Wirkungen, führen.

Überraschend wurde nun gefunden, daß die Verwendung von Alkylglycosiden als Potenzierungsmittel in alkohol-oder carbonsäurehaltigen antiseptischen Mitteln zu einer deutlichen Verstärkung der bakteriziden Wirkung der Alkohole bzw. Carbonsäuren führt, was sich insbesondere in einer deutlichen Verbesserung der mikrobiziden Wirkung gegenüber grampositiven Bakterien zeigt. Umgekehrt konnte eine gleich gute mikrobizide Wirkung, d.h. die Abtötung bestimmter Keime, schon bei sehr viel niedrigeren Konzentrationen als diese aus dem Stand der Technik bekannt sind, erreicht werden, was unmittelbar zur Folge hat, daß die Einsatzkonzentrationen der als mikrobizide Mittel wirkenden Alko hole bzw. Carbonsäuren deutlich reduziert und damit nicht nur ökonomische Vorteile erzielt, sondern auch Nebenwirkungen bzw. Nachteile der oben genannten Art beseitigt werden können.

Die Erfindung betrifft die Verwendung von Alkylglycosiden in Abmischung mit bakterizid wirkenden Alkoholen oder Carbonsäuren in wasserhaltigen Behandlungslösungen zur Potenzierung der mikrobiziden Wirkung dieser Verbindungen.

Die Erfindung betrifft außerdem wasserhaltige Desinfektions-und Reinigungsmittel mit bakterizider Wirkung, die durch einen Gehalt an Wirkstoffgemischen aus Alkylglycosiden und bakteriziden Alkoholen oder Carbonsäuren gekennzeichnet sind.

Mikrobizide Wirkstoffe für die Abmischung mit Alkylglycosiden sind im Rahmen der erfindungsgemäßen Verwendung Alkohole oder Carbonsäuren, wobei aus den jeweiligen Gruppen bakterizid wirksame Verbindungen einzeln eingesetzt oder auch mehrere Verbindungen der gleichen Klasse miteinander gemischt werden können.

Als bakterizid bekannte Alkohole sind dabei aliphatische und phenylaliphatische, d.h. durch Phenylgrup-

2

EP 0 252 276 B1

pen in der aliphatischen C-Kette substituierte aliphatische Alkohole bekannt, die im Rahmen der erfindungsgemäßen Verwendung eine oder mehrere Hydroxylgruppen enthalten können.

Von den aliphatischen Alkoholen sind bei der erfindungsgemäßen Verwendung bevorzugt geradkettige oder verzweigte, unsubstituierte oder mono-oder disubsti tuierte aliphatische Alkohole, die im Alkyl-oder Alkylenrest 1 bis 6 C-Atome enthalten. Einer oder mehrere der genannten Alkohole werden, in der erfindungsgemäß angegebenen Art und Weise, mit Alkylglycosiden abgemischt. Die genannten Alkohole besitzen in eine ausgezeichnete Löslichkeit in wässrigen Medien und weisen - wie bereits bekannt ist - ausgeprägte bakterizide Eigenschaften auf. Aus der genannten Gruppe sind als besonders bevorzugt geradkettige oder verzweigte unsubstituierte aliphatische Alkohole mit 2 bis 4 C-Atomen im Alkyl-oder Alkylenrest zu nennen, wobei mit besonderem Vorteil, d.h. unter Erzielung einer besonders guten bakteriziden Wirkung, Ethanol, n-Propanol, Isopropanol oder deren Mischungen untereinander in Abmischung mit Alkylglycosiden verwendet werden können.

Es ist jedoch auch möglich, anstelle der genannten Alkohole oder in Abmischung mit ihnen geradkettige oder verzweigte, mit einem oder zwei Substituenten aus der Gruppe Cl, Br oder $NO_2$ substituierte aliphatische Alkohole mit 2 bis 4 C-Atomen als bakterizid wirksame Substanzen einzusetzen. Aus der Gruppe dieser Verbindungen ist wegen seiner guten bakteriziden Eigenschaften 2-Bromo-2-nitro-1.3-propandiol besonders hervorzuheben, das im Handel unter der Bezeichnung "Bronopol" angeboten wird.

In einer weiteren Ausführungsform der Erfindung ist es auch möglich, anstelle rein aliphatischer Alkohole auch geradkettige oder verzweigte, unsubsitutierte oder mono-oder disubstituierte phenylaliphatische Alkohole mit 1 bis 3 C-Atomen im Alkylenrest als bakterizid wirksame Alkohole zu verwenden. Im Rahmen der vorliegenden Offenbarung werden unter "phenylalipha tische" Alkohole solche Alkohole verstanden, in denen die Alkoholfunktion an die Alkylkette gebunden ist und der Alkylrest zusätzlich einen Phenylrest als Substituenten enthält. Aus der Gruppe derartiger Alkohole werden in Abmischung mit Alkylglycosiden insbesondere geradkettige, unsubstituierte oder mit einem oder zwei Substituenten aus der Gruppe Cl, Br oder $NO_2$ substituierte phenylaliphatische Alkohole mit 1 bis 3 C-Atomen im Alkylenrest bevorzugt. Als besonders vorteilhaft bakterizid wirksamer Alkohol dieser Klasse ist beispielsweise Benzylalkohol zu nennen, da er eine ausgezeichnete bakterizide Wirkung schon in verhältnismäßig kleinen Anwendungskonzentrationen zeigt.

Entsprechend der erfindungsgemäßen Verwendung können auch Carbonsäuren, die antimikrobiell wirksam sind, mit Alkylglycosiden abgemischt werden. Auch hierbei ist die Verwendung einer antimikrobiell wirksamen Carbonsäure in Abmischung mit den Alkylglycosiden oder, alternativ dazu, auch die Verwendung mehrerer Carbonsäuren in Abmischung mit den Alkylglycosiden möglich.

Im Zuge der erfindungsgemäßen Verwendung kommen dabei insbesondere aliphatische oder aromatische, eine oder mehrere Carboxylgruppen enthaltende Carbonsäuren oder deren wasserlösliche Salze in Abmischungen mit den Alkylglycosiden zum Einsatz. Bevorzugt sind dabei geradkettige oder verzweigte, gesättigte oder ungesättigte, unsubstituierte oder mono-oder disubstituierte aliphatische Mono-oder Dicarbonsäuren mit 1 bis 12 C-Atomen, einzeln oder in Mischungen miteinander, wobei im Rahmen der erfindungsgemäßen Verwendung wiederum auch deren wasserlösliche Salze verwendet werden können. Aus der genannten Gruppe werden aufgrund ihrer guten Wasserlöslichkeit und der - an sich bekannten - antimikrobiellen Wirkung geradkettige oder verzweigte unsubstituierte aliphatische Monocarbonsäuren mit 3 bis 6 C-Atomen und deren wasserlösliche Salze bevorzugt, wobei mit besonderem Vorteil, d.h. mit guter bakterizider Wirkung schon bei vergleichsweise niedrigen Konzentrationen, Propionsäure, Buttersäure, Valeriansäure oder deren wasserlösliche Salze in Abmischungen mit den Alkylglycosiden verwendet werden.

Es ist jedoch auch möglich, eine oder mehrere Carbonsäuren - oder deren wasserlösliche Salze - aus der Gruppe geradkettiger oder verzweigter, einfach oder mehrfach ungesättigter, aliphatischer Monocarbonsäuren mit 3 bis 6 C-Atomen in Abmischungen mit Alkylglycosiden erfindungsgemäß zu verwenden. Mit besonderem Vorteil werden aus dieser Gruppe Sorbinsäure oder deren wasserlösliche Salze in Abmischungen mit Alkylglycosiden verwendet, wobei auch die bakterizide bzw. konservierende Wirkung von Sorbinsäure an sich bekannt ist.

Weiterhin können geradkettige oder verzweigte, gesättigte oder ungesättigte aliphatische Carbonsäuren mit 3 bis 6 C-Atomen, die mit einem oder zwei Substituenten aus der Gruppe Cl, Br, $NO_2$ und OH substituiert sind, oder deren wasserlösliche Salze, in Abmischungen mit Alkylglykosiden verwendet werden. In dieser Gruppe ist Milchsäure von besonderer Bedeutung.

Als weitere vorteilhafte Ausführungsform der Erfindung ist es anzusehen, als bakterizid wirkende Verbindungen unsubstituierte oder mit einem oder zwei Substituenten aus der Gruppe Cl, Br und OH subsitutierte, einringige aromatische Carbonsäuren oder deren Salze in Abmischun gen mit Alkylglycosiden zu verwenden. Auch innerhalb dieser Ausführungsform können die genannten Verbindungen einzeln oder in

3

beliebigen Mischungen miteinander in Abmischungen mit den Alkylglycosiden verwendet werden. Als bevorzugt sind aus der Gruppe dieser Verbindungen Benzoesäure, Salicylsäure und deren wasserlösliche Salze anzusehen, die - wie an sich bekannt - schon in vergleichsweise geringen Konzentrationen bakterizid wirksam sind und auch in entsprechenden antimikrobiell wirkenden Mitteln eingesetzt werden.

Die im Rahmen der erfindungsgemäß verwendbaren antimikrobiell wirksamen Carbonsäuren genannten, ebenfalls verwendbaren wasserlöslichen Salze sind in erster Linie Alkalimetallsalze, wobei von diesen in der Praxis bevorzugt die Natriumsalze aufgrund ihrer leichten Zugänglichkeit verwendet werden.

Der überraschende Vorteil der erfindungsgemäßen Verwendung von Alkylglycosiden in Abmischung mit den genannten, bakterizid wirkenden Alkoholen oder Carbonsäuren ist darin zu sehen, daß bei üblichen Einsatzmengen der antimikrobiell wirkenden Verbindungen die mikrobizide Wirkung durch Zusatz von Alkylglycosiden sehr viel schneller erreicht wird oder daß, alternativ, die üblichen Einsatzmengen zum Erreichen einer gleich guten bakteriziden Wirkung deutlich gesenkt werden können. Unter dem zuletzt genannten Aspekt der Erfindung wird es in überraschender Weise möglich, die Einsatzmengen für die praktische Anwendung deutlich zu reduzieren und dabei zu erreichen, daß die ökonomischen und durch die Verwendung der jeweiligen Mittel bedingten Nachteile, wie deren hoher Dampfdruck, die mit den Verbindungen verbundene Geruchsbelästigung usw., in einfacher Weise beseitigt werden. Zudem ist es möglich, durch die Verwendung der Alkylglycoside, die als solche biologisch leicht abbaubar sind, gezielt die antimikrobielle Wirkung der Alkohole bzw. Carbonsäuren zu verstärken bzw. zu potenzieren, wobei die Wirkung anderer Bestandteile in keiner Weise beeinflußt wird.

Werden die Alkylglykoside unter dem zuerst genannten Aspekt der Erfindung zur Beschleunigung der antimikrobiellen Wirkung der bakterizid wirkenden Alkohole und Carbonsäuren eingesetzt, so kommen die genannten Alkohole und Carbonsäuren in den an sich bekannten und üblichen Konzentrationen zur Anwendung. Sollen dagegen die Alkylglykoside nach dem oben erwähnten zweiten Aspekt der Erfindung eingesetzt werden, so können die bei der Anwendung von bakterizid wirksamen Alkoholen und Carbonsäuren üblichen Konzentrationen beträchtlich unterschritten werden. Zahlenmäßig können diese Einsatzkonzentrationen - entsprechend der sehr unterschiedlichen Wirksamkeit der bakterizid wirksamen Komponenten - in weiten Grenzen schwanken, so daß für die bakterizid wirksamen Komponenten Konzentrationen von 0,01 bis 30 Gew.-%, bezogen auf die gesamte Behandlungslösung, in Betracht kommen.

Die bevorzugten aliphatischen Monoalkohole wie Ethanol n-Propanol und Isopropanol werden insbesondere in Konzentrationen von 8 bis 30 Gew.-%, bezogen auf die gesamte Behandlungslösung eingesetzt. Stärker bakterizid wirksame Alkohole, beispielsweise Benzylalkohol kommen bevorzugt in Konzentrationen von 1 bis 3 Gew.-% und bakterizid wirksame Carbonsäuren bevorzugt in Konzentrationen von 0,01 bis 3 Gew.-%, jeweils bezogen auf die gesamte Behandlungslösung zur Anwendung.

Die erfindungsgemäß als Potenzierungsmittel eingesetzten Alkylglycoside sind aus dem Stand der Technik, beispielsweise aus den eingangs genannten Druckschriften, bekannt. Sie werden in ebenfalls bekannter Weise aus Fettalkoholen und Zuckern durch direkte Umsetzung hergestellt. Im Rahmen der erfindungsgemäßen Verwendung in Abmischung mit den bakterizid wirkenden Alkoholen oder Carbonsäuren finden ein oder mehrere Alkylglycoside Anwendung, die im Saccharidteil aus einem Glycosidrest (Alkylmonoglycoside) oder mehreren Glycosidresten (Alkyloligoglycoside) bestehen. Bei den erfindungsgemäß verwendeten Alkylglycosiden werden bis zu 8 Saccharidreste glycosidisch miteinander verbunden und die daraus entstehenden Alkylmono-oder -oligoglycoside mit bakterizid wirkenden Alkoholen oder Carbonsäuren der oben genannten Art abgemischt. Bevorzugt finden dabei Alkylmonoglycoside mit 1 bis 3 Saccharidresten, die glycosidisch miteinander verbunden sind. Verwendung. Dabei können dies beliebige Saccharidreste sein; bevorzugt werden jedoch Alkylglycoside verwendet, die 1 bis 3 Glucose-oder Maltosereste im Glycosidteil enthalten.

Im Alkylteil der erfindungsgemäß in Abmischung mit bakterizid wirkenden Alkoholen oder Carbonsäuren verwendeten Alkylglycoside finden sich Alkylreste mit 1 bis 18 C-Atomen, wie sie von synthetischen und/oder aus nativen Quellen stammenden Fettalkoholen bekannt sind. Bevorzugt findet man Alkylreste mit 6 bis 18 C-Atomen. Die diesen Alkylglycosiden zugrundeliegenden Fettalkohole stehen in preiswerter Form aus nativen Quellen zur Verfügung.

Bei der Angabe der erfindungsgemäß in Abmischung mit den bakterizid wirkenden Alkoholen oder Carbonsäuren verwendeten Alkylglycoside ist die Angabe der Zahl der Saccharidreste - wie üblich - als ein statistischer Mittelwert anzusehen, dem die bei derartigen Produkten übliche Verteilung zugrundeliegt. Im Rahmen der erfindungsgemäßen Verwendung sind Alkylglycoside mit 10 bis 14 C-Atomen im Alkylrest und mit bis zu 2 Glycosidresten, vorzugsweise mit bis zu 1,5 Glycosidresten, besonders geeignet.

Bei der Verwendung der Alkylglycoside in Abmischung mit den bakterizid wirkenden Alkoholen oder Carbonsäuren liegen die zur Potenzierung der mikrobiziden Wirkung der genannten Verbindungen erfindungsgemäß verwendeten Konzentrationen der Alkylglycoside im Bereich von 10 bis 2000 ppm, bevorzugt im Bereich von 50 bis 500 ppm, bezogen auf die gesamte Behandlungslösung. In bestimmten Abmischungen können jedoch auch sehr geringe Konzentrationen, beispielsweise 10 ppm, in Verbindung mit entsprechenden bakterizid wirkenden Alkoholen oder Carbonsäuren zu einer deutlichen Wirkungsverstärkung führen. Für den Einsatz der genannten antimikrobiellen Wirkstoffe auf wichtigen Anwendungsgebieten erschließt sich damit die Möglichkeit, schon bei geringen Mengen an Alkylglycosiden als Potenzierungsmittel mit deutlich verringerten Wirkstoffgehalten arbeiten zu können und gleichwohl eine befriedigende mikrobizide Wirkung zu erzielen.

Dies zeigt sich insbesondere im Bereich der Bekämpfung grampositiver Keime. Es hat sich gezeigt, daß in Desinfektions-und Reinigungsmitteln mit bakterizider Wirkung gegen grampositive Keime eine besonders deutliche Wirkungssteigerung der antiseptischen Mittel zu erzielen ist. Dies spielt eine besondere Rolle auf dem Gebiet der Mittel zur Körperpflege. Als solche kommen in erster Linie Zahnpasten, Zahnpulver und Mundwässer in Frage. Es können jedoch auch andere Desinfektions-und Reinigungsmittel in ihrer bakteriziden Wirkung deutlich verstärkt werden.

Gerade auf diesem Gebiet kommt der Vorteil der erfindungsgemäßen Verwendung von Alkylglycosiden in Abmischung mit bakterizid wirkenden Alkoholen oder Carbonsäuren zur Potenzierung der mikrobiziden Wirkung der genannten Verbindungen besonders gut zum tragen, da die in derartigen Mitteln, beispielsweise Zahnpasten, zugelassenen bakteriostatischen Mittel in den erlaubten Konzentrationen eine echte anti-Plaque-Wirkung derzeit nicht erzielen können. Es ist jedoch bekannt, daß bei der Bildung des Zahnbelags und der dadurch mit ausgelösten Kariesentwicklung grampositive Bakterien eine besondere Rolle spielen und dabei deren Bekämpfung besondere Aufmerksamkeit geschenkt wird. Die erfindungsgemäße Möglichkeit der Wirkungsverstärkung gerade gegenüber den grampositiven Bakterien erschließt hier den Zugang zu weitaus wirkungsvolleren Mitteln als sie bisher zur Verfügung standen, ohne daß es dazu einer Erhöhung der Wirkstoffkonzentration bedarf.

Die erfindungsgemäßen Desinfektions-und Reinigungsmittel mit bakterizider Wirkung, die durch einen im oben genannten Bereich liegenden Gehalt an Wirkstoffgemischen aus Alkylglycosiden der genannten Art und bakteriziden Alkoholen oder Carbonsäuren gekennzeichnet sind, weisen im Normalfall einen pH-Wert im Bereich von 4,5 bis 9,5 auf; ein bevorzugter Bereich liegt zwischen 6 und 8. Die erfindungsgemäß verwendete Wirkstoffkombination aus Alkylglycosiden und bakterizid wirkenden Alkoholen oder Carbonsäuren wird gege benenfalls zusammen mit geeigneten Träger-und Hilfsmaterialien eingesetzt, die ihrerseits an sich bekannte Funktionen derartiger Desinfektions-oder Reinigungsmittel übernehmen. Im Einzelfall kann die Trägerkomponente eines derartigen Mund-und/oder Zahnpflegemittels oder ähnlichen Desinfektions-und Reinigungsmittels eine übliche Zahnpaste, Mundspülzubereitung, wässrige Lösung, Kaugummimasse, ein Gelee oder dergleichen sein.

Beispielsweise enthalten Zahnpflege-bzw. Zahnputzmittel ein schleifend bzw. scheuernd wirkendes Poliermittel und normalerweise Schaumbildner, Geschmacksstoffe und Süßmittel. Zahnpasten enthalten außerdem meist Feuchthaltemittel, Bindemittel und Wasser. Bekannte geeignete Poliermittel sind beispielsweise Calciumcarbonat, Dicalciumorthophosphat-dihydrat, Calciumpyrophosphat, Calciumpolymetaphosphat und unlösliches Natriumpolymetaphosphat, Aluminiumtrihydroxid, alpha-Aluminiumoxid und Kieselsäuren, insbesondere Gelkieselsäuren und Fällungskieselsäuren. Die Verwendung von Schleif-bzw. Scheuermitteln, die mit den bakterizid wirkenden Alkoholen bzw. Carbonsäuren kompatibel sind, kann besonders bevorzugt sein. Auch hierzu wird auf die Angaben des Standes der Technik verwiesen, wie sie beispielsweise in der DE-OS 26 27 548 angegeben sind.

Der Gesamtanteil an Schleif-bzw. Scheuermitteln kann in den als Zahnputzmittel geeigneten erfindungsgemäßen Desinfektions-und Reinigungsmitteln mit bakterizider Wirkung etwa 0,5 bis 95 % des Gewichts des gesamten Mittels betragen. Üblicherweise liegt ihr Anteil bei Zahnpasten im Bereich von 6 bis 60 Gew.-%, bei Zahnpulvern im Bereich von 20 bis 95 %.

Zahnpflege-bzw. Zahnputzmittel enthalten üblicherweise Tenside als Schaumbildner. Es können dazu übliche, nicht-seifenartige, nichtionische, kationischen, zwitterionische und amphotere organische synthetische Tenside verwendet werden. Besonders geeignet sind jedoch die erfindungsgemäß als Wirkungsverstärker verwendeten Alkylglycoside, deren Tensideigenschaften ebenfalls aus dem Stand der Technik bekannt sind. Geeignete nichtionische Detergentien sind Kondensationsprodukte von Alkylenoxiden mit organischen hydrophoben Verbindungen, die beispielsweise einen aliphatischen oder alkylaromatischen Rest enthalten. Schaumbildner werden, soweit sie eingesetzt werden, in Zahnputz-bzw. Zahnpflegemitteln üblicherweise in Mengen von 0,5 bis 5 Gew.-% verwendet.

Sofern erwünscht, können auch den erfindungsgemäßen Desinfektions-und Reinigungsmitteln, insbesondere den für die Zahn-bzw. Mundpflege geeigneten Mitteln geeignete Geschmacksstoffe zugesetzt werden. Derartige Geschmacksstoffe sind beispielsweise Methylsalicylat, Pfefferminzöl, Sassafras-Öl und Anisöl. Geschmacksstoffe werden üblicherweise in Mengen von 0,01 bis 2,0 Gew.-% verwendet. In Ergänzung dazu bzw. stattdessen können gegebenenfalls auch Süßmittel bzw. Süßstoffe verwendet werden, deren Menge üblicherweise im Bereich von 0,05 bis 2 Gew.-% liegt.

Ebenfalls möglicherweise mitverwendbare Verdickungsmittel sind die üblichen Verdickungsmittel wie Hydroxyethylcellulose und wasserlösliche Salze von Celluloseethern, natürlichen Gummen bzw. Pflanzenschleimen. Verwendbar sind auch kolloidale anorganische Komponenten, wie feinzerteiltes Siliciumdioxid oder kolloidales Magnesiumaluminiumsilikat. Für die möglicherweise in Betracht zu ziehende Mitverwendung derartiger Substanzen wird auf den bereits zitierten druckschriftlichen Stand der Technik verwiesen. Die Mengen an Verdickungsmitteln liegen üblicherweise im Bereich von 0,1 bis 5,0 Gew.-% des jeweiligen Mittels, beispielsweise der Zahnpasta. Sofern erwünscht, können auch Feuchthaltemittel zugesetzt werden. Als solche geeignet sind beispielsweise Glycerin, Sorbit und andere mehrwertige Alkohole sowie deren Mischungen. Sie können in Mengen von etwa 1 bis 50 Gew.-% des jeweiligen Mittels, beispielsweise der Zahnpastenzubereitung, vorliegen und sind üblicherweise mit Wasser abgemischt.

Mundspülmittel enthalten meist eine Wasser-/Ethylalkohol-Lösung und gewünschtenfalls weitere Komponenten, wie Geschmacksstoffe, Süßmittel und Feuchthaltemittel der erwähnten Art. Im erfindungsgemäßen Sinn enthalten sie die geschilderte Kombination von antimikrobiell wirksamen Alkoholen oder Carbonsäuren und Alkylglycosiden, wobei die Wirkkomponenten in den oben genannten Mengen vorliegen.

Die Erfindung wird durch die nachfolgenden Beispiele näher erläutert.

## A. Mikrobizide Wirksamkeit

Die mikrobizide Wirksamkeit der erfindungsgemäß verwendeten Abmischungen aus bakterizid wirksamen Alkoholen oder Carbonsäuren und Alkylglycosiden wurde gegenüber folgenden Testkeimsuspensionen bestimmt:

a) Staphylococcus aureus: $2 \times 10^9$ Keime/ml;

b) Streptococcus mutans: $1 \times 10^9$ Keime/ml;

c) Escherichia coli: $2 \times 10^9$ Keime/ml;

d) Candida albicans: $2 \times 10^8$ Keime/ml.

Die Abtötungszeiten der zu untersuchenden Kombinationen wurden mit Hilfe des Suspensionstests ermittelt. Unter Verwendung von Wasser einer Härte von 17 °dH wurden Testlösungen hergestellt, die die in den Tabellen angegebenen Mengen an Alkylglycosid sowie die ebenfalls angegebenen Mengen an bakterizid wirkendem Alkohol bzw. an Carbonsäure enthielten. Daneben wurden Vergleichslösungen hergestellt, die einerseits nur bakterizid wirkende Verbindungen in den angegebenen Konzentrationen und andererseits nur Alkylglycoside in einer Konzentration von 10 000 ppm enthielten.

Bei Raumtemperatur wurden jeweils 0,1 ml Testkeimsuspension in Reagenzgläser pipettiert und mit jeweils 10 ml der oben beschriebenen Test-oder Vergleichslösungen vermischt. Nach unterschiedlichen Einwirkungszeiten (bis zu 60 min) wurden den Reagenzgläsern mit Hilfe einer Impföse ca. 0,05 ml Material entnommen und auf einem Nähragar, das als Enthemmer 3 % Tween 80 und 0,3 % Lecithin enthielt, ausgestrichen. Das Nährmedium bestand für die Keime (a) bis (d) aus 2,5 gew.-%iger Standard-I-Bouillon (Merck) und für den Keim (e) aus Würze-Bouillon pH 5 (Merck), die zur Verfestigung jeweils 1,2 Gew.-% Agar enthielten. Die Proben wurden bei 37°C bzw. 30°C bebrütet. Nach frühestens 3 Tagen wurden die Kulturen makroskopisch auf Wachstum beurteilt und auf diesem Weg die Abtötungszeit oder der Restkeimgehalt ermittelt.

In den nachfolgenden Tabellen bedeuten:

" + " weniger als 50,

" +  + " weniger als 200,

" +  +  + " mehr als 200 Restkeime nach 60 Minuten Einwirkungszeit.

## Beispiel 1

Zur Feststellung der Steigerung der mikrobiziden Wirkung von Alkoholen durch eine Abmischung mit

A. $C_{12/14}$-Alkyl-oligoglukosid (Oligomerisierungsgrad 1,5), dessen Alkylreste sich von einem n-Dodecanol/n-Tetradecanolgemisch im Gewichtsverhältnis 70 : 30 ableiten bzw.

B. Undecenyl-monoglucosid in Mengen von jeweils 0,100 und 1000 ppm wurde ein halbquantitativer Suspensionstest durchgeführt. Die Konzentrationen an mikrobizidem Wirkstoff sind Spalte 2 der nachfolgenden Tabellen 1 und 2 zu entnehmen. Die mikrobizide Wirkung der Alkohole wurde an Staphylococcus aureus (a), Escherichia coli (d) und Candida albicans (e) geprüft. Die Ergebnisse (Abtötungszeiten bzw. Restkeimgehaltes nach 60 Minuten der Einwirkung der Wirkstoffkombination) sind den nachfolgenden Tabellen 1 und 2 zu entnehmen.

# Tabelle 1

## Suspensionstest halbquantitativ

Steigerung der mikrobiziden Wirkung von Alkoholen durch Kombination mit Alkylglycosid (A)

| Wirkstoff | Wirkstoff-konz. (%) | Staph. aureus Glucosidkonzentration | | | E. coli Glucosidkonzentration | | | Cand. albicans Glucosidkonzentration | | |
|---|---|---|---|---|---|---|---|---|---|---|
| | | 0ppm | 100ppm | 1000ppm | 0ppm | 100ppm | 1000ppm | 0ppm | 100ppm | 1000ppm |
| Ethanol | 30 | 60 | $\leq$ 5 | $\leq$5 | 60 | $\leq$ 5 | $\leq$5 | 15 | 15 | 60 |
| | 25 | 60 | $\leq$5 | $\leq$5 | ++ | 60 | 60 | + | 15 | 60 |
| | 20 | +++ | 15 | $\leq$5 | ++ | ++ | +++ | +++ | + | + |
| n-Propanol | 20 | 15 | $\leq$5 | $\leq$5 | 15 | $\leq$5 | $\leq$5 | 15 | $\leq$5 | $\leq$5 |
| | 16 | 15 | $\leq$5 | $\leq$5 | 15 | $\leq$5 | $\leq$5 | 60 | $\leq$5 | $\leq$5 |
| | 12 | +++ | $\leq$5 | $\leq$5 | +++ | 60 | $\leq$5 | +++ | 15 | 15 |
| i-Propanol | 20 | 15 | $\leq$5 | $\leq$5 | 15 | $\leq$5 | 15 | 60 | $\leq$5 | $\leq$5 |
| | 16 | 15 | $\leq$5 | 15 | 60 | 60 | 60 | + | 15 | 15 |
| | 12 | 60 | 15 | 15 | +++ | +++ | +++ | +++ | ++ | + |
| Benzylal-kohol | 2,5 | $\leq$5 | $\leq$5 | $\leq$5 | $\leq$5 | $\leq$5 | 15 | 60 | 15 | $\leq$5 |
| | 2,0 | $\leq$5 | $\leq$5 | 15 | $\leq$5 | $\leq$5 | 15 | +++ | + | ++ |
| | 1,5 | 60 | 15 | 15 | 60 | 60 | +++ | +++ | +++ | +++ |

Zahlen in der Tabelle = Abtötungszeit in Minuten

+, ++, +++ = steigender Restkeimgehalt nach 60 Minuten Einwirkung

EP 0 252 276 B1

Tabelle 2

Suspensionstest halbquantitativ

Steigerung der mikrobiziden Wirkung von Alkoholen durch Kombination mit Alkylglycosid (B)

| Wirkstoff | Wirkstoff-konz. (%) | Staph. aureus Glucosidkonzentration | | | E. coli Glucosidkonzentration | | | Cand. albicans Glucosidkonzentration | | |
|---|---|---|---|---|---|---|---|---|---|---|
| | | 0ppm | 100ppm | 1000ppm | 0ppm | 100ppm | 1000ppm | 0ppm | 100ppm | 1000ppm |
| Ethanol | 30 | 60 | ≤5 | ≤5 | 60 | 15 | ≤5 | 15 | 15 | 15 |
| | 25 | 60 | ≤5 | ≤5 | ++ | + | 15 | + | 60 | 15 |
| | 20 | +++ | 15 | ≤5 | ++ | ++ | 60 | +++ | +++ | 60 |
| n-Propanol | 20 | 15 | ≤5 | ≤5 | 15 | ≤5 | ≤5 | 15 | ≤5 | ≤5 |
| | 16 | 15 | ≤5 | ≤5 | 15 | ≤5 | ≤5 | 60 | ≤5 | ≤5 |
| | 12 | +++ | ≤5 | ≤5 | +++ | ≤5 | ≤5 | +++ | 15 | ≤5 |
| i-Propanol | 20 | 15 | ≤5 | ≤5 | 15 | ≤5 | ≤5 | 60 | ≤5 | ≤5 |
| | 16 | 15 | ≤5 | ≤5 | 60 | 15 | 15 | + | 60 | 15 |
| | 12 | 60 | ≤5 | 15 | +++ | +++ | ++ | +++ | + | + |
| Benzylal-kohol | 2,5 | ≤5 | ≤5 | ≤5 | ≤5 | ≤5 | ≤5 | 60 | 15 | 60 |
| | 2,0 | ≤5 | ≤5 | ≤5 | ≤5 | ≤5 | ≤5 | +++ | ++ | 60 |
| | 1,5 | 60 | 60 | ≤5 | 60 | 60 | 60 | +++ | +++ | +++ |

Zahlen in der Tabelle = Abtötungszeit in Minuten

+, ++, +++ = steigender Restkeimgehalt nach 60 Minuten Einwirkung

Ergebnis:

Wie sich den Tabellen 1 und 2 entnehmen läßt, ergeben sich bei der Verwendung von Alkylglycosiden in Abmischungen mit Alkoholen deutliche Wirkungssteigerungen hinsichtlich der mikrobiziden Wirkung der jeweiligen Alkohole. Diese zeigen sich am deutlichsten für Ethanol und n-Propanol.

Beispiel 2

Zur Überprüfung der Steigerung der mikrobiziden Wirkung organischer Carbonsäuren durch Abmischung mit den Alkylglycosiden

A. $C_{12/14}$-Alkyl-oligoglucosid (Oligomerisierungsgrad 1,4) dessen Alkylreste sich von einem n-Dodecanol/n-Tetradecanolgemisch im Gewichtsverhältnis 70 : 30 und

B. Undecenyl-monoglucosid wurde ein halbquantitativer Suspensionstest durchgeführt, in dem die mikrobizide Wirkung der Abmischungen gegenüber Staphylococcus aureus (a), Escherichia coli (d) und Candida albicans (e) überprüft wurde. Die Konzentrationen der mikrobizid wirkenden Carbonsäuren sind Spalte 2 der Tabellen 3 und 4 zu entnehmen; die eingesetzten Carbonsäuren waren Benzoesäure, Milchsäure, Propionsäure-Natriumsalz, Salicylsäure und Sorbinsäure. Die Ergebnisse sind den nachfolgenden Tabellen 3 und 4 zu entnehmen.

## Tabelle 3

### Suspensionstest halbquantitativ

Steigerung der mikrobiziden Wirkung organischer Säuren durch Kombination mit Alkylglycosid (A)

| Wirkstoff | Wirk-stoff-konz. (%) | pH-Wert | Staph. aureus Glucosidkonzentration | | | E. coli Glucosidkonzentration | | | Cand. albicans Glucosidkonzentration | | |
|---|---|---|---|---|---|---|---|---|---|---|---|
| | | | 0ppm | 100ppm | 1000ppm | 0ppm | 100ppm | 1000ppm | 0ppm | 100ppm | 1000ppm |
| Benzoesäure | 1,0 | 2,9 | 15 | ≦5 | ≦5 | + | ≦5 | 15 | 60 | 15 | 15 |
| | 0,3 | 2,9 | 60 | ≦5 | ≦5 | +++ | 15 | 15 | +++ | + | +++ |
| | 0,1 | 2,9 | + | ≦5 | 15 | +++ | ++ | ++ | +++ | ++ | +++ |
| Milchsäure | 2,0 | 1,9 | 60 | | | 60 | | | +++ | | |
| | 1,0 | 2,1 | + | ≦5 | 60 | + | 15 | 60 | +++ | +++ | +++ |
| | 0,5 | 2,4 | ++ | 15 | 60 | +++ | 60 | + | +++ | +++ | +++ |
| | 0,1 | 2,7 | | 60 | 60 | | ++ | ++ | | | |
| Salicylsäure | 1,0 | 2,4 | ≦5 | ≦5 | ≦5 | 15 | ≦5 | 60 | 15 | ≦5 | 15 |
| | 0,3 | 2,4 | ≦5 | ≦5 | ≦5 | 60 | ≦5 | 60 | 15 | ≦5 | 15 |
| | 0,1 | 2,4 | ≦5 | ≦5 | ≦5 | 60 | ≦5 | + | 60 | 15 | 15 |
| Sorbinsäure | 2,0 | 2,4 | 60 | ≦5 | ≦5 | ++ | 60 | 15 | +++ | ++ | +++ |
| | 1,0 | 2,6 | 60 | ≦5 | ≦5 | ++ | + | 60 | +++ | +++ | +++ |
| | 0,5 | 2,8 | + | ≦5 | ≦5 | +++ | ++ | + | +++ | +++ | +++ |

Zahlen in der Tabelle = Abtötungszeiten in Minuten

+, ++, +++ = zunehmender Restkeimgehalt nach 60 Minuten Einwirkung

### Tabelle 4

### Suspensionstest halbquantitativ

Steigerung der mikrobiziden Wirkung organischer Säuren durch Kombination mit Undecenylglucosid (B)

| Wirkstoff | Wirkstoffkonz. (%) | pH-Wert | Staph. aureus 0ppm | 100ppm | 1000ppm | E. coli 0ppm | 100ppm | 1000ppm | Cand. albicans 0ppm | 100ppm | 1000ppm |
|---|---|---|---|---|---|---|---|---|---|---|---|
| Benzoesäure | 1,0 | 2,9 | 15 | 60 | ≤5 | + | ≤5 | ≤5 | 60 | 60 | 15 |
| | 0,3 | 2,9 | 60 | 60 | ≤5 | ++ | 15 | ≤5 | + | +++ | + |
| | 0,1 | 2,9 | + | 60 | ≤5 | ++ | + | ≤5 | +++ | +++ | + |
| Milchsäure | 2,0 | 1,9 | +++ | | | 15 | | | + | | |
| | 1,0 | 2,1 | +++ | ≤5 | | + | 15 | | +++ | +++ | +++ |
| | 0,5 | 2,4 | +++ | + | ≤5 | ++ | + | | +++ | +++ | +++ |
| | 0,1 | 2,7 | | ++ | 15 | | ++ | 15 | | +++ | +++ |
| | 0,05 | | | | 60 | | | + | | | |
| | 0,025 | 3,1 | | | 60 | | | +++ | | | |
| Salicylsäure | 1,0 | 2,4 | ≤5 | ≤5 | ≤5 | 60 | ≤5 | ≤5 | 15 | ≤5 | ≤5 |
| | 0,3 | 2,4 | 60 | ≤5 | ≤5 | 60 | ≤5 | ≤5 | 15 | ≤5 | ≤5 |
| | 0,1 | 2,4 | 60 | 15 | 15 | 60 | 15 | 15 | 60 | 15 | ≤5 |
| Sorbinsäure | 2,0 | 2,4 | 15 | 15 | ≤5 | + | 60 | ≤5 | +++ | +++ | 60 |
| | 1,0 | 2,6 | 60 | 60 | ≤5 | ++ | 60 | ≤5 | +++ | +++ | 60 |
| | 0,5 | 2,8 | + | 60 | ≤5 | +++ | + | ≤5 | +++ | +++ | ++ |

Zahlen in der Tabelle = Abtötungszeiten in Minuten

+, ++, +++ = zunehmender Restkeimgehalt nach 60 Minuten Einwirkung

Ergebnis:

Wirkungssteigerungen sind bei allen genannten Carbonsäuren festzustellen, wobei Steigerungsfaktoren bis zu 10 beobachtet werden.

### Beispiel 3

Der halbquantitative Suspensionstest des Beispiels 2 wurde mit Benzoesäure und Milchsäure als mikrobizid wirkenden Carbonsäuren gegenüber Staphylococcus aureus (a) und Escherichia coli (e) wiederholt, wobei zu Beispiel 2 unterschiedliche Wirkstoffkonzentrationen in den Abmischungen eingesetzt wurden. Die Ergebnisse sind der nachfolgenden Tabelle 5 zu entnehmen.

## Tabelle 5

### Halbquantitativer Suspensionstest

Wirkstoffe in Kombination mit 1000 ppm Undecenylglucosid (B) bzw. ohne Glucosid

| Wirkstoff | Wirkstoffkonz. (%) | Staph. aureus Glucosidkonz. | | E. coli Glucosidkonz. | |
|---|---|---|---|---|---|
| | | 0ppm | 1000ppm | 0ppm | 1000ppm |
| Benzoesäure | 0,2 | 60 | 5 | ++ | 5 |
| | 0,1 | +++ | 5 | +++ | 15 |
| | 0,05 | +++ | 5 | +++ | 60 |
| Milchsäure | 0,5 | +++ | 5 | +++ | 5 |
| | 0,25 | +++ | 5 | +++ | 5 |
| | 0,1 | +++ | 15 | +++ | 5 |
| Kontrolle ohne Wirkstoff | – | +++ | ++ | +++ | +++ |

Zahlen in der Tabelle = Abtötungszeit in Minuten.
+, ++, +++ = zunehmender Restkeimgehalt nach 60 Minuten Einwirkung.

Ergebnis:

Auch bei Konzentrationen der mikrobiziden Wirkstoffe in den in Tabelle 5 angegebenen Bereichen führte der Zusatz von 1000 ppm des Glucosids zu einer beträchtlichen Steigerung der mikrobiziden Wirksamkeit von Benzoesäure bzw. Milchsäure. Während ohne Glucosidzusatz bei 2000 bzw. 5000 ppm allenfalls schwache mikrobizide Effekte der Benzoesäure bzw. der Milchsäure erkennbar sind, wird durch Zusatz von 1000 ppm des Glucosids schon bei Bakterizidkonzentrationen von 500 bis 1000 ppm eine befriedigende Abtötung der Prüfkeime bewirkt.

## Patentansprüche

1. Verwendung von Alkylmonoglycosiden mit 1 bis 18 C-Atomen in den Alkylresten und/oder Alkyloligoglycosiden mit bis zu 8 glycosidisch gebundenen Saccharidresten und 1 bis 18 C-Atomen in den Alkylresten in Abmischung mit

    a) bakterizid wirkenden aliphatischen oder phenylaliphatischen, eine oder mehrere Hydroxylgruppen enthaltenden, gegebenenfalls mit einem oder mehreren Substituenten aus der Gruppe Cl, Br und NO$_2$ substituierten Alkoholen oder

    b) bakterizid wirkenden aliphatischen und/oder aromatischen, eine oder mehrere Carboxylgruppen enthaltenden, gegebenenfalls mit einem oder zwei Substituenten aus der Gruppe Cl, Br oder OH substituierten Carbonsäuren oder deren wasserlöslichen Salzen

in wasserhaltigen Behandlungslösungen zur Potenzierung der mikrobiziden Wirkung dieser Verbindungen, wobei die Behandlungslösungen 10 bis 2000 ppm, bezogen auf die gesamte Behandlungslösung und 1 bis 30 Gew.-% aliphatische oder phenylaliphatische Alkohole oder 0,01 bis 3 Gew.-% jeweils bezogen auf die gesamte Behandlungslösung --aliphatische und/oder aromatische Carbonsäuren enthält.

2. Verwendung nach Anspruch 1, dadurch gekennzeichnet, daß die Alkylglycoside mit einem oder mehreren geradkettigen oder verzweigten, unsubstituierten oder monosubstituierten oder disubstituierten aliphatischen Alkoholen mit 1 bis 6 C-Atomen im Alkyl- oder Alkylenrest abgemischt werden.

3. Verwendung nach Anspruch 2, dadurch gekennzeichnet, daß die Alkylglycoside mit einem oder mehreren geradkettigen oder verzweigten unsubstituierten aliphatischen Alkoholen mit 2 bis 4 C-Atomen im Alkyl- oder Alkylenrest abgemischt werden.

4. Verwendung nach Anspruch 3, dadurch gekennzeichnet, daß die Alkylglycoside mit einem oder mehreren Alkoholen aus der Gruppe Ethanol, n-Propanol und Isopropanol abgemischt werden.

5. Verwendung nach Anspruch 2, dadurch gekennzeichnet, daß die Alkylglycoside mit einem oder mehreren geradkettigen oder verzweigten, mit einem oder mehreren Substituenten aus der Gruppe Cl, Br und NO$_2$ substituierten aliphatischen Alkoholen mit 2 bis 4 C-Atomen abgemischt werden.

6. Verwendung nach Anspruch 5, dadurch gekennzeichnet, daß die Alkylglycoside mit 2-Bromo-2-nitro-1.3-propandiol abgemischt werden.

7. Verwendung nach Anspruch 1, dadurch gekennzeichnet, daß die Alkylglycoside mit einem oder mehreren geradkettigen oder verzweigten, unsubstituierten, mono- oder disubstituierten phenylaliphatischen Alkoholen mit 1 bis 3 C-Atomen im Alkylenrest abgemischt werden.

8. Verwendung nach Anspruch 7, dadurch gekennzeichnet, daß die Alkylglycoside mit einem oder mehreren geradkettigen, unsubstituierten oder mit einem oder zwei Substituenten aus der Gruppe Cl, Br und NO$_2$ substituierten phenylaliphatischen Alkoholen mit 1 bis 3 C-Atomen abgemischt werden.

9. Verwendung nach Anspruch 8, dadurch gekennzeichnet, daß die Alkylglycoside mit Benzylalkohol abgemischt werden.

10. Verwendung nach Anspruch 1, dadurch gekennzeichnet, daß die Alkylglycoside mit einer oder mehreren geradkettigen oder verzweigten, gesättigten oder ungesättigten, unsubstituierten oder mono- oder disubstituierten aliphatischen Monocarbonsäuren oder Dicarbonsäuren mit 1 bis 12 C-Atomen oder deren wasserlöslichen Salzen abgemischt werden.

11. Verwendung nach Anspruch 10, dadurch gekennzeichnet, daß die Alkylglycoside mit einer oder mehreren geradkettigen oder verzweigten, unsubstituierten aliphatischen Monocarbonsäuren mit 3 bis 6 C-Atomen oder deren wasserlöslichen Salzen abgemischt werden.

12. Verwendung nach Anspruch 11, dadurch gekennzeichnet, daß die Alkylglycoside mit Propionsäure, Buttersäure, Valeriansäure und/oder deren wasserlöslichen Salzen abgemischt werden.

13. Verwendung nach Anspruch 10, dadurch gekennzeichnet, daß die Alkylglycoside mit einer oder mehreren geradkettigen oder verzweigten, einfach oder mehrfach ungesättigten aliphatischen Monocarbonsäuren mit 3 bis 6 C-Atomen und/oder deren wasserlöslichen Salzen abgemischt werden.

**14.** Verwendung nach Anspruch 13, dadurch gekennzeichnet, daß die Alkylglycoside mit Sorbinsäure und/oder deren wasserlöslichen Salzen abgemischt werden.

**15.** Verwendung nach Anspruch 10, dadurch gekennzeichnet, daß die Alkylglycoside mit einer oder mehreren geradkettigen oder verzweigten gesättigten oder ungesättigten, mit einem oder zwei Substituenten aus der Gruppe Cl, Br und OH substituierten aliphatischen Monocarbonsäure und/oder deren wasserlöslichen Salzen abgemischt werden.

**16.** Verwendung nach Anspruch 15, dadurch gekennzeichnet, daß die Alkylglycoside mit Milchsäure und/oder deren Salzen abgemischt werden.

**17.** Verwendung nach Anspruch 1, dadurch gekennzeichnet, daß die Alkylglycoside mit unsubstituierten oder mit einem oder zwei Substituenten aus der Gruppe Cl, Br und OH substituierten einringigen aromatischen Carbonsäuren und/oder deren Salzen abgemischt werden.

**18.** Verwendung nach Anspruch 17, dadurch gekennzeichnet, daß die Alkylglycoside mit Benzoesäure oder Salicylsäure oder deren wasserlöslichen Salzen abgemischt werden.

**19.** Verwendung nach Anspruch 1 und 10 bis 18, dadurch gekennzeichnet, daß als wasserlösliche Carbonsäuresalze die Natriumsalze verwendet werden.

**20.** Verwendung nach Ansprüchen 1 bis 19, dadurch gekennzeichnet, daß als Alkylglycoside ein oder mehrere Alkyloligoglycoside mit 1 bis 3 glycosidisch verbundenen Saccharidresten mit bakterizid wirkenden Alkoholen oder Carbonsäuren abgemischt werden.

**21.** Verwendung nach Anspruch 20, dadurch gekennzeichnet, daß als Alkylglycoside solche mit 1 bis 3 Glucose- oder Maltoseresten verwendet werden.

**22.** Verwendung nach Ansprüchen 1 bis 21, dadurch gekennzeichnet, daß als Alkylglycoside solche mit 1 bis 18 C-Atomen in den Alkylresten verwendet werden.

**23.** Verwendung nach Anspruch 22, dadurch gekennzeichnet, daß Alkylglycoside solche mit 6 bis 18 C-Atomen im Alkylrest verwendet werden.

**24.** Verwendung nach Ansprüchen 1 bis 23 dadurch gekennzeichnet, daß die Konzentration an Alkylclycosiden im Bereich von 50 bis 500 ppm, bezogen auf die gesamte Behandlungslösung liegen.

**25.** Verwendung nach den Ansprüchen 1 bis 24, dadurch gekennzeichnet, daß die wasserhaltige Behandlungslösung die wässrige Phase eines Mund- und/oder Zahnpflegemittels ist.

**Claims**

**1.** The use of alkyl monoglycosides containing 1 to 18 C atoms in the alkyl radicals and/or alkyl oligoglycosides containing up to 8 glycosidically bonded saccharide units and 1 to 18 C atoms in the alkyl radicals in admixture with

a) bactericidally active aliphatic or phenyl-aliphatic alcohols containing one or more hydroxyl groups and optionally substituted by one or more substituents from the group consisting of Cl, Br and $NO_2$ or

b) bactericidally active aliphatic and/or aromatic carboxylic acids containing one or more carboxyl groups and optionally substituted by one or two substituents from the group consisting of Cl, Br or OH or water-soluble salts thereof

in water-containing treatment solutions for potentiating the microbicidal effect of those compounds, the treatment solutions containing 10 to 2,000 ppm, based on the treatment solution as a whole, and 1 to 30% by weight aliphatic or phenyl-aliphatic alcohols or 0.01 to 3% by weight, based on the treatment solution as a whole, aliphatic and/or aromatic carboxylic acids.

**2.** The use claimed in claim 1, characterized in that the alkyl glycosides are mixed with one or more straight-chain or branched, unsubstituted or monosubstituted or disubstituted aliphatic alcohols containing from 1 to 6 carbon atoms in the alkyl or alkylene radical.

3. The use claimed in claim 2, characterized in that the alkyl glycosides are mixed with one or more straight-chain or branched, unsubstituted aliphatic alcohols containing from 2 to 4 carbon atoms in the alkyl or alkylene radical.

4. The use claimed in claim 3, characterized in that the alkyl glycosides are mixed with one or more alcohols from the group consisting of ethanol, n-propanol and isopropanol.

5. The use claimed in claim 2, characterized in that the alkyl glycosides are mixed with one or more straight-chain or branched aliphatic alcohols containing 2 to 4 carbon atoms substituted by one or more substituents from the group consisting of Cl, Br and $NO_2$.

6. The use claimed in claim 5, characterized in that the alkyl glycosides are mixed with 2-bromo-2-nitro-1,3-propanediol.

7. The use claimed in claim 1, characterized in that the alkyl glycosides are mixed with one or more straight-chain or branched, unsubstituted, mono- or di-substituted phenyl-aliphatic alcohols containing 1 to 3 carbon atoms in the alkylene radical.

8. The use claimed in claim 7, characterized in that the alkyl glycosides are mixed with one or more straight-chain, unsubstituted phenyl-aliphatic alcohols containing 1 to 3 carbon atoms or phenyl-aliphatic alcohols containing 1 to 3 carbon atoms substituted by one or two substituents from the group cosisting of Cl, Br and $NO_2$.

9. The use claimed in claim 8, characterized in that the alkyl glycosides are mixed with benzyl alcohol.

10. The use claimed in claim 1, characterized in that the alkyl glycosides are mixed with one or more straight-chain or branched, saturated or unsaturated, unsubstituted or mono- or disubstituted aliphatic monocarboxylic acids of dicarboxylic acids containing from 1 to 12 carbon atoms or water-soluble salts thereof.

11. The use claimed in claim 10, characterized in that the alkyl glycosides are mixed with one or more straight-chain or branched, unsubstituted aliphatic monocarboxylic acids containing from 3 to 6 carbon atoms or water-soluble salts thereof.

12. The use claimed in claim 11, characterized in that the alkyl glycosides are mixed with propionic acid, butyric acid, valeric acid and/or water-soluble salts thereof.

13. The use claimed in claim 10, characterized in that the alkyl glycosides are mixed with one or more straight-chain or branched, mono- or polyunsaturated aliphatic mono-carboxylic acids containing from 3 to 6 carbon atoms and/or water-soluble salts thereof.

14. The use claimed in claim 13, characterized in that the alkyl glycosides are mixed with sorbic acid and/or water-soluble salts thereof.

15. The use claimed in claim 10, characterized in that the alkyl glycosides are mixed with one or more straight-chain or branched, saturated or unsaturated aliphatic mono-carboxylic acids substituted by one or two substituents from the group consisting of Cl, Br and OH and/or water-soluble salts thereof.

16. The use claimed in claim 15, characterized in that the alkyl glycosides are mixed with lactic acid and/or salts thereof.

17. The use claimed in claim 1, characterized in that the alkyl glycosides are mixed with unsubstituted, monocyclic aromatic carboxylic acids or with monocyclic aromatic carboxylic acids substituted by one or two substituents from the group consisting of Cl, Br and OH and/or salts thereof.

18. The use claimed in claim 17, characterized in that the alkyl glycosides are mixed with benzoic acid or salicylic acid or water-soluble salts thereof.

**19.** The use claimed in claims 1 and 10 to 18, characterized in that sodium salts are used as the water-soluble carboxylic acid salts.

**20.** The use claimed in claims 1 to 19, characterized in that one or more alkyl oligoglycosides containing 1 to 3 glycosidically bonded saccharide units are mixed as alkyl glycosides with bactericidally active alcohols or carboxylic acids.

**21.** The use claimed in claim 20, characterized in that the alkyl glycosides used contain from 1 to 3 glucose or maltose units.

**22.** The use claimed in claims 1 to 21, characterized in that the alkyl glycosides used contain from 1 to 18 carbon atoms in the alkyl radicals.

**23.** The use claimed in claim 22, characterized in that the alkyl glycosides used contain from 6 to 18 carbon atoms in the alkyl radical.

**24.** The use claimed in claims 1 to 23, characterized in that the concentration of alkyl glycosides is in the range of from 50 to 500 ppm, based on the treatment solution as a whole.

**25.** The use claimed in claims 1 to 24, characterized in that the water-containing treatment solution is the aqueous phase of an oral hygiene and/or dental care preparation.

## Revendications

**1.** Utilisation d'alkylmonoglycosides ayant de 1 à 18 atomes de carbone dans las radicaux alkyle et/ou d'alkyloligoglycosides ayant jusqu'à 8 radicaux saccharides à liaison glycosidique et ayant de 1 à 18 atomes de carbone dans les radicaux alkyle en mélange avec :

   a) des alcools aliphatiques ou phénylaliphatiques à effet bactéricide contenant un ou plusieurs groupes hydroxyle, substitués éventuellement par un ou plusieurs substituants choisis dans le groupe Cl, Br et $NO_2$, ou

   b) des acides carboxyliques aliphatiques et/ou aromatiques à action bactéricide contenant un ou plusieurs groupes carboxyle, substitués éventuellement par un ou deux substituants choisis dans le groupe Cl, Br ou OH ou leurs sels solubles dans l'eau

   en solutions aqueuses de traitement pour potentialiser l'effet microbicide de ces composés, tandis que les solutions de traitement contiennent de 10 à 2000 ppm rapportées à la solution de traitement et de 1 à 30% en poids d'alcools aliphatiques ou phénylaliphatiques ou 0,01 à 3% en poids rapportés chaque fois au total de la solution de traitement d'acides carboxyliques aliphatiques et/ou aromatiques.

**2.** Utilisation selon la revendication 1, caractérisée en ce que les alkylglycosides sont mélangés avec un ou plusieurs alcools aliphatiques non substitués ou monosubstitués ou disubstitués à chaîne linéaire ou ramifiée ayant de 1 à 6 atomes de carbone dans le radical alkyle ou alkylène.

**3.** Utilisation selon la revendication 2, caractérisée en ce que les alkylglycosides sont mélangés avec un ou plusieurs alcools aliphatiques non substitués à chaîne droite ou ramifiée ayant de 2 à 4 atomes de carbone dans le radical alkyle ou alkylène.

**4.** Utilisation selon la revendication 3, caractérisée en ce que les alkylglycosides sont mélangés avec un ou plusieurs alcools choisis dans le groupe de l'éthanol, du n-propanol et de l'isopropanol.

**5.** Utilisation selon la revendication 2, caractérisée en ce que les alkylglycosides sont mélangés avec un ou plusieurs alcools aliphatiques à chaîne linéaire ou ramifiée substitués par un ou plusieurs substituants choisis dans le groupe Cl, Br et $NO_2$ et ayant de 2 à 4 atomes de carbone.

**6.** Utilisation selon la revendication 5, caractérisée en ce que les alkylglycosides sont mélangés avec du 2-bromo-2-nitro-1,3-propanediol.

7. Utilisation selon la revendication 1, caractérisée en ce que les alkylglycosides sont mélangés avec un ou plusieurs alcools phénylaliphatiques non substitués, mono- ou disubstitués à chaîne linéaire ou ramifiée ayant de 1 à 3 atomes de carbone dans le radical alkylène.

8. Utilisation selon la revendication 7, caractérisée en ce que les alkylglycosides sont mélangés avec un ou plusieurs alcools phénylaliphatiques à chaîne linéaire non substitués ou substitués par un ou deux substituants choisis dans le groupe Cl, Br et $NO_2$ et ayant de 1 à 3 atomes de carbone.

9. Utilisation selon la revendication 8, caractérisée en ce que les alkylglycosides sont mélangés avec de l'alcool benzylique.

10. Utilisation selon la revendication 1, caractérisée en ce que les alkylglycosides sont mélangés avec un ou plusieurs acides monocarboxyliques aliphatiques saturés ou non saturés, non substitués ou mono- ou disubstitués à chaîne linéaire ou ramifiée ou avec des acides dicarboxyliques ayant de 1 à 12 atomes de carbone ou avec leurs sels solubles dans l'eau.

11. Utilisation selon la revendication 10, caractérisée en ce que les alkylglycosides sont mélangés avec un ou plusieurs acides monocarboxyliques aliphatiques non substitués à chaîne linéaire ou ramifiée ayant de 3 à 6 atomes de carbone ou avec leurs sels solubles dans l'eau.

12. Utilisation selon la revendication 11, caractérisée en ce que les alkylglycosides sont mélangés avec l'acide propionique, l'acide butyrique, l'acide valérique et/ou leurs sels solubles dans l'eau.

13. Utilisation selon la revendication 10, caractérisée en ce que les alkylglycosides sont mélangés avec un ou plusieurs acides monocarboxyliques aliphatiques insaturés une ou plusieurs fois, avec chaîne linéaire ou ramifiée ayant de 3 à 6 atomes de carbone et/ou avec leurs sels solubles dans l'eau.

14. Utilisation selon la revendication 13, caractérisée en ce que les alkylglycosides sont mélangés avec l'acide sorbique et/ou ses sels solubles dans l'eau.

15. Utilisation selon la revendication 10, caractérisée en ce que les alkylglycosides sont mélangés avec un ou plusieurs acides monocarboxyliques aliphatiques saturés ou insaturés à chaîne linéaire ou ramifiée, substitués par un ou deux substituants choisis dans le groupe Cl, Br et OH et/ou avec leurs sels solubles dans l'eau.

16. Utilisation selon la revendication 15, caractérisée en ce que les alkylglycosides sont mélangés avec l'acide lactique et/ou ses sels.

17. Utilisation selon la revendication 1, caractérisée en ce que les alkylglycosides sont mélangés avec des acides carboxylique aromatiques monocycliques non substitués ou substitués par un ou deux substituants choisis dans le groupe Cl,Br et OH et/ou avec leurs sels.

18. Utilisation selon la revendication 17, caractérisée en ce que les alkylglycosides sont mélangés avec l'acide benzoïque ou l'acide salicylique ou avec leurs sels solubles dans l'eau.

19. Utilisation selon las revendications 1 et 10 à 18, caractérisée en ce que l'on utilise les sels sodiques des acides carboxyliques comme sels solubles dans l'eau.

20. Utilisation selon les revendications 1 à 19, caractérisée en ce que les alkylglycosides mélangés à des alcools ou des acides carboxyliques à action bactéricide sont un ou plusieurs alkyloligoglycosides avec des radicaux saccharides ayant de 1 à 3 liaisons glycosidiques.

21. Utilisation selon la revendication 20, caractérisée en ce que l'on utilise comme alkylglycosides ceux avec de 1 à 3 radicaux glucose ou maltose.

22. Utilisation selon les revendications 1 à 21, caractérisée en ce que l'on utilise comme alkylglycosides ceux ayant de 1 à 18 atomes de carbone dans les radicaux alkyle.

**23.** Utilisation selon la revendication 22, caractérisée en ce que l'on utilise comme alkylglycosides ceux ayant de 6 à 18 atomes de carbone dans le radical alkyle.

**24.** Utilisation selon les revendications 1 à 23, caractérisée en ce que la concentration en alkylglycosides est comprise dans la gamme de 50 à 500 ppm rapportés au total de la solution de traitement.

**25.** Utilisation selon les revendications 1 à 24, caractérisée en ce que la solution de traitement aqueuse est la phase aqueuse d'un produit pour l'entretien de la bouche et/ou des dents.